**Europäisches Patentamt**

**European Patent Office**

**Office européen des brevets**

(19)

(11) Numéro de publication : **0 115 976**
**B1**

(12) **FASCICULE DE BREVET EUROPÉEN**

(45) Date de publication du fascicule du brevet :
26.04.89

(51) Int. Cl.⁴ : **A 61 K 9/54**

(21) Numéro de dépôt : **84400033.1**

(22) Date de dépôt : **06.01.84**

(54) **Médicament à libération programmée à base d'acide acétylsalicylique.**

(30) Priorité : 28.01.83 FR 8301576

(43) Date de publication de la demande :
15.08.84 Bulletin 84/33

(45) Mention de la délivrance du brevet :
26.04.89 Bulletin 89/17

(84) Etats contractants désignés :
**AT BE CH DE FR GB IT LI LU**

(56) Documents cités :
**WO-A-82/01468**
**FR-A- 2 390 959**
**FR-A- 2 454 804**
**Le dossier contient des informations techniques
présentées postérieurement au dépôt de la demande
et ne figurant pas dans le présent fascicule.**

(73) Titulaire : **SANOFI, Société dite:**
**40, Avenue George V**
**F-75008 Paris (FR)**

(72) Inventeur : **Chick, Jacques Alexandre**
**2 Rue des Fontaines**
**F-92310 Sevres (FR)**

(74) Mandataire : **Polus, Camille et al**
**c/o Cabinet Lavoix 2, Place d'Estienne d'Orves**
**F-75441 Paris Cedex 09 (FR)**

## EP 0 115 976 B1

### Description

L'acide acétylsalicylique (ASA) est connu depuis plusieurs décennies pour ses propriétés analgésiques, antipyrétiques, anti-inflammatoires et uricosuriques. C'est aussi un antiagrégant plaquettaire à faible dose. Il est indiqué dans les douleurs et états fébriles, dans les affections rhumatismales et dans la prévention des accidents thrombo-emboliques.

Néanmoins, il peut provoquer divers effets indésirables et, en particulier, des saignements digestifs occultes et des hémorragies. L'ASA est très peu soluble en phase aqueuse. Il se disperse peu dans le contenu gastrique. Il en résulte de fortes concentrations en un petit nombre de points de la muqueuse gastrique, ce qui favorise l'apparition d'une gastrite hémorragique. Certes, celle-ci est d'une pathogénie complexe, mais pour l'essentiel, elle résulte d'un effet direct de contact de l'ASA avec la muqueuse gastrique. C'est la raison pour laquelle différentes formes galéniques ont été proposées pour éviter ces graves inconvénients et améliorer la tolérance digestive de l'ASA.

C'est ainsi que sont commercialisés des comprimés entériques, des comprimés à enrobage gastro-résistant et entéro-soluble, des comprimés effervescents tamponnés, des comprimés à croquer, des sachets-dose, etc... les doses unitaires habituelles étant de l'ordre de 100 mg pour les formes pédiatriques et comprises entre 500 mg et 1 g pour les formes orales adultes.

Toutes ces formes galéniques présentent néanmoins des inconvénients. C'est le cas, par exemple, des formes à enrobage gastro-résistant et entéro-soluble qui modifient la biodisponibilité du principe actif et de ses métabolites : le taux plasmatique n'est atteint que trois heures après la prise et la courbe des taux plasmatiques est étalée, ce qui a pour conséquence d'imposer, pour obtenir le même effet, une posologie plus forte que celle de l'ASA ordinaire. C'est le cas, également, des comprimés effervescents tamponnés car la solubilisation a des conséquences pharmacocinétiques importantes : d'une part, la résorption digestive est plus rapide et le pic plasmatique de salicylémie est atteint en moins d'une heure, deux fois plus vite qu'avec l'ASA ordinaire, d'autre part, l'élimination urinaire débute aussi plus rapidement. De plus, de telles formes impliquent l'incorporation de fortes doses d'éléments sodés pouvant être néfastes pour de nombreux sujets.

L'objet de la présente demande est une composition médicamenteuse à base d'acide acétylsalicylique comprenant un grain support d'environ 500 à 600 μm de diamètre en matière non toxique, recouvert de plusieurs couples (a) d'un revêtement constitué d'un mélange contenant de l'acide acétylsalicylique, du polyvidone-excipient et du phtalate d'éthyle, protégé par (b) un enrobage en polymère anionique de l'acide méthacrylique, (a) et (b) étant séparés par des couches de talc, le polyvidone excipient et le phtalate d'éthyle représentant 4 à 8 % du poids total de la préparation, les grains support et l'enrobage représentant de 20 à 30 % du poids total de la préparation, le nombre de couples de revêtement (a) et d'enrobage (b) étant tel que 4 % du poids de l'acide acétylsalicylique soit libéré en milieu duodénal artificiel en 1 heure et 100 % en 4 heures, qui permet une libération programmée du principe actif en milieu duodénal et qui pallie les inconvénients décrits ci-dessus, en particulier ceux liés à la tolérance gastrique.

Pour obtenir les microgranules d'ASA à libération programmée, on utilise généralement la technique déjà décrite par la demanderesse dans le brevet français N° 7 910 620 ; on fixe le principe actif sur des grains support d'environ 500 à 600 microns de diamètre, ces derniers étant obtenus à partir d'une semence de saccharose et par enrobages successifs à l'aide d'une solution hydroalcoolique composée de saccharose, et par enrobages successifs à l'aide d'une solution hydroalcoolique composée de saccharose, de polyvidone-excipient, d'alcool industriel pharmaceutique et d'eau. On saupoudre à l'aide de talc et d'amidon de maïs. Après avoir laissé sécher, on calibre et on ne retient que les grains d'environ 500 à 600 μm de diamètre. On fixe ensuite l'ASA par poudrage et pulvérisation d'une solution hydroalcoolique de polyvidone-excipient et de phtalate d'éthyle. On laisse sécher, on calibre et on vérifie la teneur en principe actif. On enrobe les grains ainsi obtenus à l'aide d'une solution de polymère anionique de l'acide méthacrylique dans l'alcool industriel pharmaceutique : on saupoudre avec du talc et on recommence ces opérations jusqu'à ce que le programme de libération recherché corresponde à la cinétique de dissolution telle que décrite ci-dessus. Les microgranules ainsi terminées sont soumises à un nouveau contrôle analytique et sont ensuite réparties dans des gélules de manière telle que chaque gélule titre 330 mg d'ASA, le poids du contenu de chaque gélule étant de 440 mg environ. Ce titre en ASA n'est donné qu'à titre illustratif non limitatif puisque des essais identiques ont été réalisés avec une quantité de principe actif équivalent, par exemple, à 165 mg. Il va de soi que la quantité d'ASA peut varier en fonction des nécessités thérapeutiques et de l'utilisation médicale dudit principe actif.

Il est donné, ci-après un exemple illustrant les quantités de produits nécessaires à la réalisation des gélules de l'invention.

On réalise les grains support dans les proportions suivantes, les quantités étant rapportées à 1 kg de grains support :

(Voir tableau page 3)

2

| Composition | Gamme générale grammes | Gamme préférée grammes |
|---|---|---|
| Saccharose | 300 à 750 | 694,6 |
| Amidon de maïs | 100 à 150 | 138,8 |
| Talc | 100 à 150 | 138,8 |
| Polyvidone-excipient | 25 à 30 | 27,8 |

les solvants intermédiaires étant l'alcool industriel pharmaceutique et l'eau. Après séchage et calibrage, les grains obtenus ont un diamètre de 500 μm environ.

Ces grains support sont ensuite enrobés à l'aide d'une solution hydroalcoolique de polyvidone-excipient et de phtalate d'éthyle, contenant l'ASA, ce qui permet de fixer le principe actif sur les grains supports de manière telle que pour 440 mg de contenu de gélule, cette quantité titre 330 mg d'ASA. On laisse sécher, on calibre et on dose le principe actif de préférence par voie spectrophotométrique à 530 nm comparativement à une solution témoin d'ASA. On enrobe ces grains à l'aide d'une solution alcoolique de polymère anionique de l'acide méthacrylique ; on poudre avec du talc dans la proportion d'environ 1/10 par rapport à la masse totale et on recommence les opérations jusqu'à ce que le programme de libération soit respecté. Les grains support et l'enrobage représentent environ 25 p.100 du poids total. On répartit ensuite en gélules et on procède à un nouveau dosage de l'ASA selon la même technique spectrophotométrique utilisée précédemment, le poids total contenu dans chaque gélule étant de 440 mg de grains, eux-mêmes contenant 330 mg d'ASA.

La libération du principe actif devant être obtenue en milieu duodénal, la cinétique de libération in vitro est réalisée en milieu aqueux artificiel, à pH 6,9. Cette cinétique a permis d'obtenir les résultats suivants :

après 1 heure          4 p.100
après 4 heures       100 p.100

Essais toxicologiques

Il a également été procédé à l'étude toxicologique des gélules d'ASA à libération programmée comparativement à des gélules d'ASA non programmées. Il y a lieu de préciser que ces essais ont été réalisés avec des préparations contenant soit 165 mg de principe actif par gélule, soit 330 mg d'ASA par gélule. Après pulvérisation du contenu des gélules et mise en suspension de la poudre ainsi obtenue dans une solution aqueuse de gomme arabique à 5 p.100, cette suspension a été administrée par tubage gastrique à des souris de souche Swiss également réparties entre les deux sexes, chaque dose étudiée étant administrée à des lots de 5 mâles et de 5 femelles, le poids corporel des mâles étant compris entre 23 et 25 g et celui des femelles entre 20 et 23 g. La dose maximale administrée a été de 1 000 mg d'ASA par kg de poids corporel, les animaux étant placés en observation pendant les 14 jours ayant suivi l'administration. Aucun symptôme d'intolérance n'a été observé ; l'autopsie de 5 animaux mâles et de 5 animaux femelles s'est révélée particulièrement satisfaisante. Ainsi, nulle altération lésionnelle n'a été constatée.

D'autres essais ont été réalisés avec des rats de souche SPRAGUE-DAWLEY, dans les mêmes conditions que pour les souris. Les animaux mâles pesaient 200 g et les animaux femelles 170 g. La dose administrée a également été de 1 000 mg/kg. Aucun symptôme d'intolérance n'a été observé pendant les 14 jours suivant l'administration et l'autopsie n'a rien révélé d'anormal sur le plan général et sur le plan histologique.

La tolérance chez l'animal a donc été la même, que la préparation en cause ait été ou non à libération programmée.

Essais de biodisponibilité et de tolérance gastrique

Des essais ont été réalisés chez l'homme, pour d'une part connaître la biodisponibilité de la préparation de l'invention et d'autre part, pour en étudier la tolérance gastrique.

3

1 — Les essais de biodisponibilité ont porté sur trois préparations différentes d'ASA :

la préparation de l'invention présentée sous forme de gélule,

une préparation commercialisée sous forme de comprimés et présentée comme ayant un effet retard, une préparation présentée sous forme de gélule et ne contenant que de l'acide acétylsalicylique de la pharmacopée.

La biodisponibilité a été appréciée après administration des trois préparations, chez l'homme, en cross over, en mesurant les trois paramètres suivants :

— aires sous les courbes de concentration plasmatique,

— concentrations plasmatiques maxima obtenues (C max),

— temps des concentrations plasmatiques maxima (T max).

L'étude de biodisponibilité a été réalisée sur douze sujets de sexe masculin et féminin exempts d'affections cardio-vasculaires, hépatiques, rénales ou gastro-intestinales ; ces sujets n'avaient reçu aucun médicament les jours précédents et pendant toute la durée de l'étude. Chaque sujet a reçu soit une gélule selon l'invention contenant 330 mg d'ASA, soit 1/2 comprimé de la préparation commercialisée (correspondant à 325 mg d'ASA), soit une gélule ne contenant que 330 mg d'ASA de la pharmacopée ; chacune des trois formulations a été absorbée au moins à 8 jours d'intervalle, avec 200 ml d'eau ; enfin, chaque sujet entrant dans l'étude a reçu un numéro d'ordre correspondant à la randomisation de l'administration des trois formulations suivant quatre carrés latins. Les taux de la salicylémie — qui a été dosée suivant la méthode de ROWLAND et RIEGELMAN (J. Pharm. Sci., 1976, 56, 717-738) — ont été mesurés aux temps 0, 0.5, 1, 2, 3, 4, 6, 8, 10, 12 et 24 heures après l'administration.

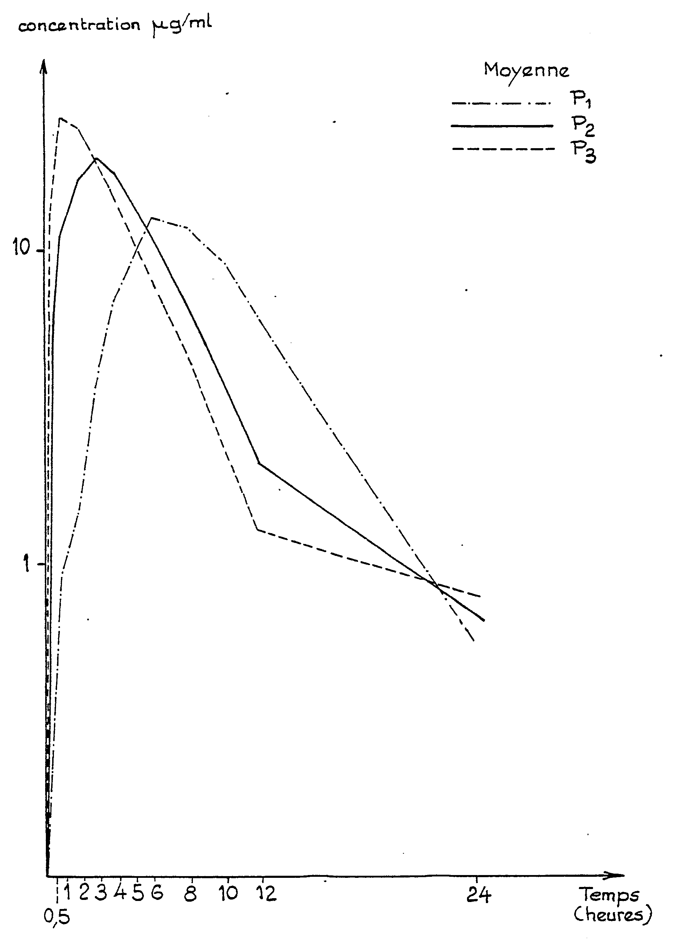

Les résultats moyens obtenus sont consignés dans le tableau ci-dessous, la figure n° 1 illustrant la concentration moyenne de la salicylémie (µg/ml) mesurée aux temps précités :

| Produits administrés | Aires sous les courbes | C max | T max |
|---|---|---|---|
| Préparation de l'invention $(P_1)$ | $129,39 \pm 9,90$ | $13,76 \pm 0,95$ | $7,00 \pm 0,46$ |
| Préparation commercialisée $(P_2)$ | $131,47 \pm 10,85$ | $19,90 \pm 1,39$ | $2,75 \pm 0,22$ |
| Préparation selon la Pharmacopée $(P_3)$ | $131,32 \pm 9,86$ | $28,15 \pm 1,23$ | $1,25 \pm 0,17$ |

Leur étude permet de formuler les commentaires suivants quant à la préparation de l'invention :

1.1. par rapport à la présentation d'ASA de la Pharmacopée, si les deux aires sous les courbes de concentration plasmatique sont semblables, traduisant une résorption équivalente, en revanche, les temps des concentrations plasmatiques maxima sont très différents, traduisant un étalement de la résorption de l'ASA contenu dans la préparation de l'invention. Correspondant à cet étalement, il existe une nette différence entre les taux plasmatiques, celui obtenu après l'administation de la préparation de l'invention étant nettement inférieur à celui obtenu après administration de la préparation selon la Pharmacopée,

1.2. par rapport à la préparation commercialisée, les résultats obtenus montrent que la préparation de l'invention présente une résorption significativement plus retardée : en effet, si les aires sous les courbes sont similaires, par contre, le temps de pic maxima est significativement plus tardif pour la préparation de l'invention (7,00 ± 0,46 heures vs 2,75 ± 0,22 heures) ; cette différence est significative au test de WILCOXON.

Ainsi donc, toutes les caractéristiques de biodisponibilité de la préparation objet de l'invention répondent effectivement aux critères d'une formulation retard.

2 — L'étude de la tolérance gastrique a été réalisée par des essais contrôlés comparatifs en double aveugle avec répartition aléatoire entre la préparation de l'invention et un produit de référence. Ce dernier, l'acétylsalicylate de lysine, a été choisi car il est réputé présenter la meilleure tolérance clinique et biologique de tous les sels d'aspirine.

Les essais ont été conduits sur 52 sujets (23 hommes et 29 femmes) âgés de 30 à 80 ans. 21 personnes ont reçu le produit de référence et 31 la préparation objet de l'invention.

La préparation de l'invention titrait 330 mg d'ASA et le produit de référence contenait 549 mg d'acétylsalicylate de lysine correspondant à 330 mg d'ASA. Les deux préparations étaient présentées sous forme de gélules.

En fonction des indications thérapeutiques traitées (syndrôme grippal, algies diverses, rééducation articulaire post-fracturaire, etc.), le nombre de gélules administrées a pu atteindre 60, le nombre de journées de traitement s'échelonnant de 2 à 9 jours ou plus.

Le tableau ci-après résume l'étude de la tolérance :

| Manifestations indésirables | Préparation de l'invention | Préparation de référence |
|---|---|---|
| Troubles gastriques importants | 0 | 4 |
| Troubles gastriques modérés | 2 | 4 |
| Troubles gastriques faibles | 5 | 4 |
| Troubles diarrhéiques faibles | 1 | 3 |
| Syndrômes hémorragiques modérés (appréciés à l'hémocult) | 0 | 2 |
| Syndrômes hémorragiques faibles (appréciés à l'hémocult) | 2 | 2 |
| TOTAUX | 10 | 19 |
| Aucune manifestation indésirable | 24 | 9 |

Il y a lieu de noter que chez aucun sujet, il n'a été constaté de manifestations tégumentaires du type érythème, purpura, prurit, etc., ou allergiques du type asthme ou rhinite. Les seules manifestations indésirables ne sont donc survenues qu'au niveau de la sphère gastrointestinale ou de la sphère hémostatique. Ces intolérances peuvent se résumer comme suit :

(Voir tableau page 6 )

| Manifestations indésira-bles chez un même sujet | Préparation de l'invention | Préparation de référence |
|---|---|---|
| à 3 niveaux (gastrique, intestinal, sanguin) | 0 | 9,5 p.100 |
| à 2 niveaux (gastrique, intestinal ou sanguin) | 9,67 p.100 | 14,28 p.100 |
| à 1 seul niveau (gastrique) | 19,20 p.100 | 33,33 p.100 |
| TOTAUX | 22,58 p.100 (7 cas) | 57,14 p.100 (12 cas) |

Ainsi donc, avec la préparation de l'invention, 24 cas, soit 77,41 p.100, n'ont pas présenté de manifestation indésirable alors qu'avec la préparation de référence, 9 cas, soit 42,85 p.100, n'ont pas présenté de manifestation indésirable.

Il apparaît ainsi que la préparation de l'invention est nettement mieux tolérée que la préparation de référence.

Il est donné ci-dessous à titre d'exemple, une formulation unitaire du médicament de l'invention :

| | |
|---|---|
| Acide acétylsalicylique | 330 mg |
| Saccharose | 38 mg |
| Amidon de maïs | 10 mg |
| Talc | 20 mg |
| Polyvidone-excipient | 37 mg |
| Polymère anionique de l'acide méthacrylique | 5,7 mg |
| Phtalate d'éthyle | 2 mg |

q.s.p. 1 gélule terminée à 440 mg environ

Compte tenu de ses caractéristiques de biodisponibilité et de son excellente tolérance, la posologie quotidienne du médicament de l'invention varie en fonction de l'affection traitée de 0,200 g à 3,300 g alors que pour l'ASA non programmée la posologie quotidienne est de 0,300 g à 5 g, soit une réduction moyenne de 30 à 40 p.100. Le médicament de l'invention possède toutes les indications de l'ASA et principalement les douleurs et les états fébriles, les algies rhumatismales et la prévention des états thrombo-emboliques.

**Revendication** (pour les Etats contractants : BE, CH, DE, FR, GB, IT, LI, LU)

Composition médicamenteuse à base d'acide acétylsalicylique comprenant un grain support d'environ 500 à 600 μm de diamètre en matière non toxique, recouvert de plusieurs couples (a) d'un revêtement constitué d'un mélange contenant de l'acide acétylsalicylique, du polyvidone-excipient et du phtalate d'éthyle, protégé par (b) un enrobage en polymère anionique de l'acide méthacrylique, (a) et (b) étant séparés par des couches de talc, le polyvidone excipient et le phtalate d'éthyle représentant 4 à 8 % du poids total de la préparation, les grains support et l'enrobage représentant de 20 à 30 % du poids total de la préparation, le nombre de couples de revêtement (a) et d'enrobage (b) étant tel que 4 % du poids de l'acide acétylsalicylique soit libéré en milieu duodénal artificiel en 1 heure et 100 % en 4 heures.

**Revendication** (pour l'Etat contractant AT)

Procédé de préparation d'une composition médicamenteuse à base d'acide acétylsalicylique comprenant un grain support d'environ 500 à 600 μm de diamètre en matière non toxique, recouvert de plusieurs couples (a) d'un revêtement constitué d'un mélange contenant de l'acide acétylsalicylique, du polyvidone-excipient et du phtalate d'éthyle, protégé par (b) un enrobage en polymère anionique de l'acide méthacrylique, (a) et (b) étant séparés par des couches de talc, le polyvidone excipient et le phtalate d'éthyle représentant 4 à 8 % du poids total de la préparation, les grains support et l'enrobage représentant de 20 à 30 % du poids total de la préparation, le nombre de couples de revêtement (a) et d'enrobage (b) étant tel que 4 % du poids de l'acide acétylsalicylique soit libéré en milieu duodénal artificiel en 1 heure et 100 % en 4 heures, caractérisé en ce que l'on prépare le grain support, que l'on enrobe, après séchage et calibrage, d'une solution hydroalcoolique de polyvidone-excipient et de phtalate d'éthyle contenant l'acide acétylsalicylique, on sèche et calibre, on enrobe le grain ainsi enrobé d'une solution alcoolique de polymère anionique de l'acide méthacrylique et on poudre avec du talc, l'opération d'enrobage étant recommencée jusqu'à ce que le programme de libération soit respecté.

**Claim** (for the Contracting States : BE, CH, DE, FR, GB, IT, LI, LU)

Pharmaceutical composition based on acetylsalicylic acid comprising support granules of a non-toxic substance, having a diameter of approximately 500 to 600 μm, covered with several layers (a) of a coating, consisting of a mixture containing acetylsalicylic acid, polyvidone as excipient and ethyl phthalate, protected by (b) a covering of anionic polymer of methacrylic acid, (a) and (b) being separated by layers of talc, the polyvidone excipient and the ethyl phthalate representing 4 to 8 % of the total weight of the preparation whilst the support granules and the covering represent 20 to 30 % of the total weight of the preparation, the number of layers of the coating (a) and of the covering (b) being such that 4 % of the weight of the acetylsalicylic acid are discharged in an artificial duodenal medium within 1 hour and 100 % thereof within 4 hours.

**Claim** (for the Contracting State AT)

Process for the preparation of a pharmaceutical composition based on acetylsalicylic acid, comprising support granules of a non-toxic substance, having a diameter of approximately 500 to 600 μm, covered with several layers (a) of a coating consisting of a mixture containing acetylsalicylic acid, polyvidone as excipient and ethyl phthalate, protected by (b) a covering of anionic polymer of methacrylic acid, (a) and (b) being separated by layers of talc, the polyvidone excipient and ethyl phthalate representing 4 to 8% of the total weight of the preparation, whilst the support granules and the covering represent 20 to 30 % of the total weight of the preparation, the number of layers of the coating (a) and of the cover (b) being such that 4 % of the weight of the acetylsalicylic acid are discharged in an artificial duodenal medium within 1 hour and 100 % thereof in 4 hours, characterised in that the granulated support is prepared, that after drying and calibration it is coated with a hydroalcoholic solution of polyvidone excipient and ethyl phthalate containing acetylsalicylic acid, that it is dried and calibrated, that the granule thus coated is covered with an alcoholic solution of anionic polymer of methacrylic acid and that it is powdered with talc, the covering process being repeated until the discharge programme is completed.

**Patentanspruch** (für die Vertragsstaaten : BE, CH, DE, FR, GB, IT, LI, LU)

Arzneimittel auf der Basis von Acetylsalicylsäure mit einem körnigen Träger mit einem Teilchendurchmesser von etwa 500 bis 600 μm aus einem nichttoxischen Material, überzogen mit mehreren Paaren (a) eines Überzugs aus einem Gemisch von Acetylsalicylsäure, Polyvidon als Grundmasse und Ethylphthalat, geschützt von (b) einer Umhüllung aus einem anionischen Methacrylsäure-Polymer, wobei (a) und (b) durch Talkumschichten getrennt sind, die Polyvidon-Grundmasse und das Ethylphthalat von 4 bis 8 % der Gesamtmasse der Zusammensetzung ausmachen, die Trägerkörner und die Umhüllung von 20 bis 30 % der Gesamtmasse der Zusammensetzung ausmachen, die Anzahl von Paaren des Überzugs (a) und der Umhüllung (b) so ist, daß 4 Masse-% der Acetylsalicylsäure in einem künstlichen Duodenalmedium in 1 h und 100% in 4 h freigesetzt werden.

**Patentanspruch** (für den Vertragsstaat AT)

Verfahren zur Herstellung eines Arzneimittels auf der Basis von Acetylsalicylsäure mit einem körnigen Träger mit einem Teilchendurchmesser von etwa 500 bis 600 μm aus einem nichttoxischen Material, überzogen mit mehreren Paaren (a) eines Überzugs aus einem Gemisch von Acetylsalicylsäure, Polyvidon als Grundmasse und Ethylphthalat, geschützt von (b) einer Umhüllung aus einem anionischen Methacrylsäure-Polymer, wobei (a) und (b) durch Talkumschichten getrennt sind, die Polyvidon-Grundmassse und das Ethylphthalat von 4 bis 8 % der Gesamtmasse der Zusammensetzung ausmachen, die Trägerkörner und die Umhüllung von 20 bis 30 % der Gesamtmasse der Zusammensetzung ausmachen, die Anzahl von Paaren des Überzugs (a) und der Umhüllung (b) so ist, daß 4 Masse-% der Acetylsalicylsäure in einem künstlichen Duodenalmedium in 1 h und 100 % in 4 h freigesetzt werden, gekennzeichnet durch Herstellung eines körnigen Trägers, der nach dem Trocknen und dem Klassieren mit einer wäßrigen, Acetylsalicylsäure enthaltenden Alkohollösung der Polyvidon-Grundmasse und des Ethylphthalats umhüllt, getrocknet und klassiert wird, Umhüllung der so überzogenen Körner mit einer Alkohollösung des anionischen Methacrylsäure-Polymers und Bestäuben mit Talkum, wobei die Umhüllungsbehandlung sooft wiederholt wird, bis das Freisetzungsprogramm eingehalten ist.